# DEMANDE DE BREVET EUROPEEN

(11) **EP 1 147 723 A1**
(43) Date de publication de la demande: **24.10.2001**
(21) Numéro de dépôt: 01500105.0
(22) Date de dépôt: 19.04.2001
(51) Int. Cl.: A45D 44/12

(54) **Protecteur pour pavillon auditif**

(30) Priorité: 19.04.2000 ES 200001075
(71) Demandeur: Robledo Santander, Erna, 08810 Sant Pere De Ribes, Barcelona (ES); Pons Pons, José, 08810 Sant Pere De Ribes, Barcelona (ES)
(72) Inventeur: Robledo Santander, Erna, 08810 Sant Pere De Ribes, Barcelona (ES); Pons Pons, José, 08810 Sant Pere De Ribes, Barcelona (ES)
(74) Mandataire: Urizar Anasagasti, Jesus Maria

(57) **Abrégé**

Protecteur pour pavillon auditiv constitué, pour cela et plus précisément, d'un corps lamellaire (1), de preference en plastique, dont le contour ressemble à celui de l'oreille ou pavillon auditif, bien que sensiblement surdimensionné par rapport à celui-ci, se fixant sur la périphérie dudit corps lamellaire, par sa face interne, d'un second corps (3) constituant avec le premier un réceptacle formellement et dimensionellement adéquat pour loger le pavillon auditif dans son interieur, ledit second corp ayant une ouverture (5) dûment positionnée pour permettre l'accès audit réceptacle de l'oreille.

## Description

### Objet de l'invention

La présente invention concerne un protecteur spécialement conçu pour isoler convenablement les pavillons auditifs de toute personne dans des conditions déterminées, comme par exemple, pour se doucher ou se laver les cheveux, chez des personnes ayant des problèmes otiques, pour protéger l'oreille lors des travaux de coiffure en évitant l'entrée des cheveux à l'intérieur, pour isoler le pavillon même lors de l'application de teinture en évitant ainsi que les produits chimiques de celle-ci n'entrent pas en contact avec la peau en pouvant provoquer des problèmes allergiques ou d'irritation, ainsi qu'éviter la saleté.

### Antécédents de l'invention

Bien que les protecteurs pour l'oreille sont connus, nous ne connaissons pas l'existence de protecteur du pavillon auditif.

En tant que protecteur pour l'oreille il existe différents types de bouchons qui sont implantés correctement dans le conduits auditif, en évitant que l'eau ou toute autre type de produit ne pénètre à l'intérieur de l'oreille en produisant des problèmes d'otites ou similaires chez des personnes déterminées.

Ces bouchons, bien qu'ils accomplissent de manière satisfaisante la fonction pour laquelle ils sont prévus, ils ne protègent que le conduit auditif, en laissant complètement libre le pavillon, aussi bien internement qu'externement, ce qui fait de lors de la réalisation de certains travaux de coiffure, comme par exemple la teinture des cheveux, ledit pavillon est salit par la teinture, occasionnellement la teinture agit négativement sur la peau, en l'irritant ou en produisant des allergies ou même encore lors d'une simple coupe de cheveux, les résidus des cheveux se déposent sur les plis internes du pavillon.

### Description de l'invention

Le protecteur proposé par l'invention résout de manière complètement satisfaisante la problématique antérieurement exposée, car en recouvrant totalement le pavillon auditif, aussi bien internement qu'externement, il protège ledit pavillon de tout agent externe, en évitant aussi l'accès à l'intérieur de l'oreille.

Le dit protecteur est constitué, pour cela et plus précisément, d'un corps lamellaire, de preference en plastique, mais il peut être de toute autre matière adéquate à la fonction protrectrice souhaitée, corps lamellaire dont le contour ressemble à celui de l'oreille ou pavillon auditif, bien que sensiblement surdimensionné par rapport à celui-ci, se fixant sur la périphérie dudit corps lamellaire, par sa face interne, d'un second corps constituant avec le premier un réceptacle formellement et dimensionellement adéquat pour loger le pavillon auditif dans son interieur, ledit second corp ayant une ouverture dûment positionnée pour permettre l'accès audit réceptacle de l'oreille.

La stabilisation adéquate du protecteur par rapport à l'oreille ou pavillon auditif, est obtenue grâce au fait que ladite ouverture est assisté par un élément élastique permettant l'agrandissement momentané de l'ouverture pendant l'action d'accouplement à l'oreille et une récupération élastique postérieure en étranglant par consécuent ladite ouverture sur le cou de l'oreille, en s'adaptant parfaitement à celui-ci et par conséquent, en provoquant une fermeture et une protection parfaite aussi bien de tout le pavillon auditif internement et externement, que du conduit auditif.

Ledit élément élastique peut en option être remplacer par un petit cordon ou tout autre élément capable d'étrangler ladite ouverture sur le cou de l'oreille.

### Description def dessins

Pour compléter la description en cours et afin d'aider à une meilleure compréhension des caractéristiques de l'invention, selon un exemple préféré de réalisation pratique de celle-ci, ladite description est accompagnée d'un jeu de dessins à caractère illustratif et non pas limitatif, nous avons représenté ce qui suit :

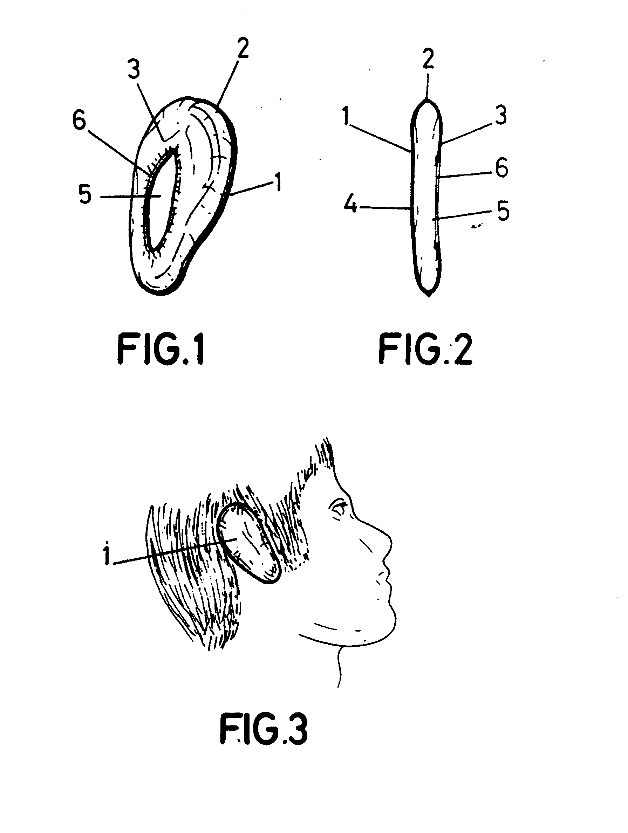

La figure 1. monte selon une représentation schématique en perspective, un protecteur pour le pavillon auditif réalisé selon l'objet de la présente invention, vu du côté de sa face interne.

La figure 2. montre un profil en section du même protecteur.

La figure 3. montre finalement une autre vue en perspective du protecteur, du côté de la face externe et dûment accouplé à une des oreilles de l'utilisateur.

### Réalisation préférée de l'invention

D'après les figures ci-dessus nous pouvons observer que le protecteur préconisé est constitué d'un corps lamellaire (1), de n'importe quel matériel imperméable, comme par exemple le plastique, dont le contour épouse formellement et dimensionnellement celui de l'oreille ou pavillon auditif de l'utilisateur, corps lamellaire (1) qui, à travers son bord périmétral (2) reçoit, au moyen de thermosoudage, soudage par ultrasons ou tout autre moyen estimé convenable, un second corps (3) ayant des caractéristiques similaires configurant avec le corps (1) un réceptacle (4) capable de logé à son intérieur l'oreille de l'utilisateur, en incorporant à cet effet, dûment positionnée une ouverture (5) dimensionnellement adéquate pour permettre le passage à travers elle de l'oreille de l'utilisateur, son embouchure étant assistée par un élément élastique (6) qui tend à étrangler ladite ouverture (5) sut le cou de l'oreille de l'utilisateur, afin de munir le protecteur de la stabilité adéquate pensant son utilisation.

L'élément élastique (6) peut être simplement fixé au corps intérieur (3), il peut être logé dans un pli tubulaire de l'embouchure de ladite ouverture (5), ou il peut même être remplacé par un petit cordon, se trouvant au sein dudit pli tubulaire, et émergeant avec ses deux extrémités à l'extérieur, celui-ci pouvant alors permettre l'étranglement de l'ouverture (5), en ayant en ce cas-ci une forme non élastique.

L'élément élastique (6) est le plus convenable pour être celui qui facilite et assouplit la manipulation de l'implantation et du désaccouplement du protecteur, bien que tout autre système d'étranglement de l'ouverture (5) assurant la consolidation du protecteur sur le cou de l'oreille soit aussi valable.

## Revendications

1. Protecteur pour le pavillon auditif, étant spécialement indiqué pour être appliqué aux personnes ayant des problèmes otiques, d'irritations ou d'allergies par rapport à certains produits, en évitant aussi bien que l'eau ou de tels produits pénètrent à l'intérieur du conduit auditif, ainsi que le contact de ceux-ci avec la peau, aussi bien de la face externe que de la face interne du pavillon, et en pouvant aussi être utilisé comme une simple barrière de retenue aussi bien des liquides que des solides, est **caractérisé en ce qu'**il est constitué d'un corps lamellaire, de n'importe quel matériel imperméable convenable, corps lamellaire qui épouse formellement et dimensionnellement la face externe du pavillon auditif'à laquelle il est destiné à s'adapter, en recevant solidairement sur sa périphérie un second corps lamellaire ayant des caractéristiques similaires, au moyen de thermosoudage, soudage par ultrasons ou tout autre moyen, les deux corps définissant un réceptacle formellement et dimensionnellement adéquat pour recevoir en son sein le pavillon auditif et le corps lamellaire interne ayant une ouverture d'accès audit réceptacle dimensionnellement adéquate pour permettre le passage à travers elle du pavillon auditif.

2. Protecteur pour le pavillon auditif, selon la revendication 1. **caractérisé en ce que** ladite ouverture du corps lamellaire interne est assistée par un élément élastique qui tend à étrangler ladite ouverture sur le cou du pavillon auditif, en ayant prévu qu'en option ledit élément élastique puisse être remplacé par un petit cordon ou tout autre élément capable d'étrangler ladite ouverture sur le cou du pavillon auditif.
